# EUROPEAN PATENT APPLICATION

(11) **EP 4 749 628 A1**
(43) Date of publication of application: **27.05.2026**
(21) Application number: 25199084.2
(22) Date of filing: 29.08.2025
(51) Int. Cl.: G16C 20/30, G16C 60/00, G16C 20/70

(54) **METHOD, PROGRAM, AND DEVICE FOR PREDICTING DELIVERY CARRIER COMPOSITION**

(30) Priority: 26.11.2024 JP 2024205588
(71) Applicant: Kabushiki Kaisha Toshiba, Kawasaki-shi, Kanagawa (JP)
(72) Inventor: ISHIHARA, Mitsuko, Kawasaki-shi (JP); OKUMURA, Shu, Kawasaki-shi (JP); FURUYA, Kozue, Kawasaki-shi (JP); NISHINO, Kaneharu, Kawasaki-shi (JP); YOSHIDA, Mitsunobu, Kawasaki-shi (JP)
(74) Representative: Marks & Clerk LLP

(57) **Abstract**

According to one arrangement, a designing method for predicting a composition of a delivery carrier that satisfies a target value of an active agent delivery amount for a target cell is provided. The method includes preparing a delivery carrier prediction tool and acquiring, using the delivery carrier prediction tool, the composition of the delivery carrier that satisfies the target value based on a lipid composition of a cell membrane of the target cell.

## Description

### FIELD

The present disclosure relates to a method, a program, and a device for predicting a delivery carrier composition.

### BACKGROUND

As a method for introducing an active agent into a cell, a method using a delivery carrier containing an active agent has been known. In such a technique, it is also known that optimal components of the delivery carrier vary for each type of cell. That is, optimizing the components of the delivery carrier may result in excellent delivery efficiency and directionality for a specific type of cell (hereinafter, referred to as "target cell"). Therefore, in order to obtain a delivery carrier suitable for the target cell, it is necessary to design the components of the delivery carrier so as to satisfy an appropriate condition for the target cell.

Designing delivery carrier according to a related art is performed by a method in which multiple types of delivery carriers with different components are produced, introducibility of the delivery carriers into a target cell is measured and confirmed, the degree of contribution of each component of the delivery carrier is estimated, and the components are adjusted based on human experience and skill. Therefore, the design technique according to the related art is very complicated and requires a large amount of trial and error, and there has been a demand for a method, a program, and a device capable of more simply designing a delivery carrier suitable for a target cell.

### BRIEF DESCRIPTION OF THE DRAWINGS

FIG. 1 is an explanatory diagram illustrating an example of a configuration of a delivery carrier composition prediction device according to an arrangement;
FIG. 2 is an explanatory diagram illustrating an example of a configuration of a target cell lipid data set;
FIG. 3 is an explanatory diagram illustrating an example of a configuration of a non-target cell lipid data set;
FIG. 4 is an explanatory diagram illustrating examples of configurations of data sets regarding an active agent delivery amount for a non-target cell, part (a) illustrating an example of a delivery carrier composition data set, and part (b) illustrating an example of a non-target cell delivery amount data set;
FIG. 5 is a flowchart illustrating an example of a designing method for a delivery carrier; and
FIG. 6 is a flowchart illustrating an example of the designing method for the delivery carrier.

### DETAILED DESCRIPTION

According to one arrangement, a designing method for a delivery carrier for acquiring a composition of the delivery carrier that satisfies a target value of a predetermined delivery amount of a delivered substance for a target cell is provided. The method includes: preparing a delivery carrier prediction tool constructed by machine learning using, as learning data sets, a delivery carrier composition data set regarding a plurality of types of delivery carriers having different component compositions, a non-target cell membrane composition data set regarding lipid compositions of cell membranes of a plurality of types of cells other than the target cell, and a non-target cell delivery amount data set regarding delivery amounts of delivered substance of the types of delivery carriers for the types of cells other than the target cell; and acquiring, using the delivery carrier prediction tool, the composition of the delivery carrier that satisfies the target value based on the lipid composition of the cell membrane of the target cell.

According to one arrangement, a program for causing a computer to predict a composition of a delivery carrier that satisfies a target value of an active agent delivery amount for a target cell is provided. The program is constructed by machine learning using, as learning data sets, a delivery carrier composition data set regarding a plurality of types of delivery carriers having different component compositions, a non-target cell membrane composition data set regarding lipid compositions of cell membranes of a plurality of types of cells other than the target cell, and a non-target cell delivery amount data set regarding delivery amounts of a delivered substance of the types of delivery carriers for the types of cells other than the target cell. The program causes the computer to predict the composition of the delivery carrier that satisfies the target value of the active agent delivery amount for the target cell based on a data set of the lipid composition of the cell membrane of the target cell.

According to one arrangement, a device for predicting a composition of a delivery carrier that satisfies a target value of an active agent delivery amount for a target cell isprovided. The device includes: an input unit configured to receive a lipid composition of a cell membrane of a target cell from outside of the device and transmit the lipid composition to a computation unit; a computation unit configured to calculate the composition of the delivery carrier that satisfies the target value of the active agent delivery amount for the target cell based on the lipid composition of the cell membrane of the target cell by using a delivery carrier prediction tool constructed by machine learning using, as learning data sets, a delivery carrier composition data set regarding a plurality of types of delivery carriers having different component compositions, a non-target cell membrane composition data set regarding lipid compositions of cell membranes of a plurality of types of cells other than a target cell, and a non-target cell delivery amount data set regarding delivery amounts of a delivered substance of the types of delivery carriers for the types of cells other than the target cell; and an output unit configured to display a computation result of the computation unit.

Hereinafter, a method, a program, and a device for predicting a delivery carrier composition according to an arrangement will be described with reference to the drawings. The drawings are schematic diagrams that illustrate the arrangement and facilitate understanding thereof, and the shapes, dimensions, proportions, and the like may be different from actual implementations. However, these can be appropriately modified based on the following description and known techniques.

### - Delivery Carrier Composition Prediction Device

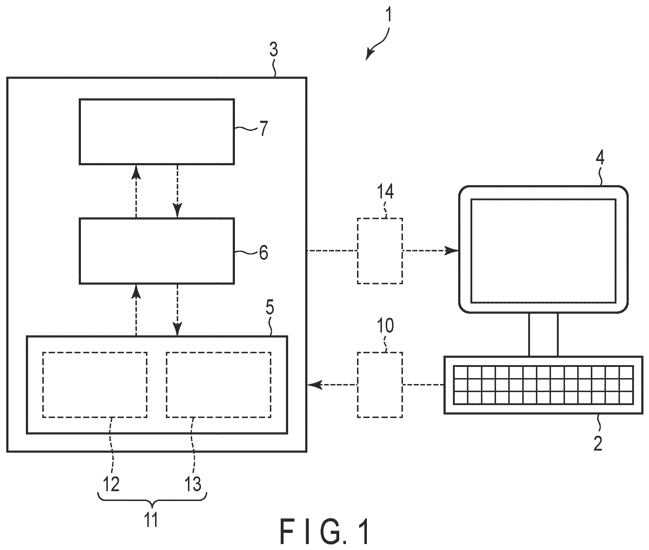

An arrangement of a delivery carrier composition prediction device 1 illustrated in FIG. 1 is used to implement a method for predicting a delivery carrier composition. The prediction device 1 and the prediction method are used to obtain a composition of a delivery carrier that satisfies a target value of a predetermined introduction rate of an active agent to a target cell. That is, according to the arrangement, a composition data set 14 (hereinafter, referred to as a "target carrier composition data set" ) of the delivery carrier that satisfies a target value of a predetermined introduction amount is acquired as described below.

The term "delivery carrier" as used herein refers to any carrier capable of delivering the active agent into various cells. The delivery carrier may be a carrier as used in general drug delivery systems.

For example, the delivery carrier is in the form of a lipid nanoparticle known to form a nano-sized capsule. Here, the lipid nanoparticle (LNP) is a substantially spherical body including a lipid membrane formed by arranging a plurality of lipid molecules by non-covalent bonds, and is, for example, a liposome, a micelle, a nanoemulsion, or a solid lipid nanoparticle. The lipid membrane may be a lipid monomolecular membrane or a lipid bilayer membrane. In addition, the lipid membrane may be formed of a single-layer membrane or may be formed of a multilayered membrane. A nucleic acid may be contained as the active agent in a central cavity of the lipid nanoparticle.

Alternatively, the delivery carrier may be in the form of a cationic polymer represented by polyethyleneimine or in the form of a cell-penetrating peptide. However, if the form in which the delivery carrier is desired to be designed has been determined, the method, the program, and the device for predicting the delivery carrier composition according to the present arrangement may be implemented using only a data set regarding the delivery carrier having the desired form.

As illustrated in FIG. 1, the prediction device 1 includes an input unit 2, a computation unit 3 connected to the input unit 2, and an output unit 4 connected to the computation unit 3. The connection between the input unit 2, the computation unit 3, and the output unit 4 may be performed by any method as long as information communication is possible.

The prediction device 1 in the present specification is defined as a computation device in which the input unit 2, the computation unit 3, and the output unit 4 are configured as separate units. However, the prediction device 1 may be interpreted as a computation system (hereinafter, referred to as a "prediction system") in which the input unit 2, the computation unit 3, and the output unit 4 are different devices or systems and which is formed by a combination of the devices or systems. That is, a prediction system having a configuration similar to that of the prediction device 1 and exhibiting a similar technical effect is also included in the scope disclosed in the present specification.

Hereinafter, each constituent element of the prediction device 1 will be described in detail. The input unit 2 is a unit that functions as an interface for reading data from the outside in the prediction device 1. As described in detail below, data (hereinafter, referred to as "input data") input to the input unit 2 includes at least a data set 10 (hereinafter, referred to as "target cell lipid data set") regarding a lipid composition of a cell membrane of the target cell. The input data may be input by a user, or may be data represented by signals or numerical values output from various units. In a case where the input data is input by the user, the input unit 2 is a user interface such as a keyboard and a mouse included in a computer.

Alternatively, the input unit 2 may be various data acquisition units, and in this case, the input data may be cell membrane lipid data measured and input using the data acquisition unit. As the data acquisition unit, various known chemical analyzers, electron microscopes, measurement devices for measuring a physical property, and the like can be used. Alternatively, in a case where the input data is input by an external data acquisition device, the input unit 2 may be a computation processing unit that executes a program for automatically performing numerical processing on a signal obtained from the external data acquisition device.

The computation unit 3 is various known computation unit groups capable of executing a predetermined program. FIG. 1 illustrates a case where the computation unit 3 is a computation unit group, and the computation unit group includes at least an auxiliary storage unit 5, a main storage unit 6, and a computation processing unit 7 connected to one another.

The auxiliary storage unit 5 is various known auxiliary storage units (for example, a hard disk drive (HDD)) and stores the predetermined program. The predetermined program includes at least a delivery carrier prediction tool (hereinafter, also simply referred to as a "prediction tool") 11 that predicts a lipid composition of the delivery carrier suitable for the lipid compositions of the cell membranes of various cells. The prediction tool is a combination of a plurality of programs, and may include a program 12 for causing the computation unit 3 to execute an algorithm (hereinafter, referred to as a "delivery amount prediction algorithm") for predicting an active agent delivery amount for the target cell by using each candidate composition data of the delivery carrier and feature amount data of the lipid composition of the target cell, and a program 13 for causing the computation unit 3 to execute an algorithm (hereinafter, referred to as a "composition optimization algorithm") for optimizing the composition of the delivery carrier based on the predicted active agent delivery amount. Furthermore, as described in detail below, the prediction tool is an artificial intelligence (AI) tool constructed by various types of machine learning using, as training data, a learning data set including data regarding a cell (hereinafter, referred to as a "non-target cell") other than the target cell. The AI tool performs training to infer a relevance between the composition of the delivery carrier, the lipid composition of the cell membrane of the cell into which the active agent is to be introduced by the delivery carrier, and the active agent delivery amount for the cell into which the active agent is to be introduced by various machine learning. The non-target cell may also include target cells cultured under different conditions, collected at different times, or obtained from different donors.

The main storage unit 6 is various known main storage units (for example, memories), the computation processing unit 7 is various known central processing units (CPU), and data processing designated by the predetermined program 12 or the like can be performed using the main storage unit 6 and the computation processing unit 7. In the prediction device **1,** the main storage unit 6 is connected to each of the auxiliary storage unit 5 and the computation processing unit 7 so as to be able to communicate with each other. Therefore, the predetermined program stored in the auxiliary storage unit 5 can be read into the main storage unit 6 and activated and can be transmitted to the computation processing unit 7 to perform the data processing, and the main storage unit 6 can receive a computation result of the computation processing unit 7. Here, the computation result of the computation processing unit 7 includes at least the target carrier composition data set 14.

The computation result obtained by the computation unit 3 is transmitted to the output unit 4. The output unit 4 is a unit or a device that functions as an interface for outputting the target carrier composition data set 14 to the outside of the prediction device 1 and displaying the target carrier composition data set 14, and is, for example, a display or the like.

### (Data Set)

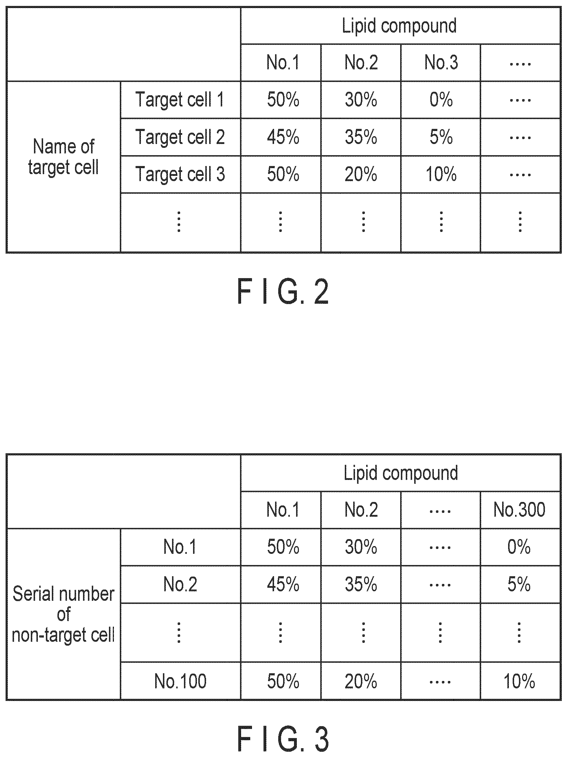

Various pieces of input data input to the prediction device 1 via the input unit 2 are described in detail below. In FIGS. 2 to 4, each data set is illustrated in a table format for description, but each data set may have any data format or data structure as long as the data format or data structure has various data elements described below. For example, the data set may be matrix data or vector data. In this case, each label of row data and column data in FIGS. 2 to 4 may be configured in a data set as data separate from the matrix data and the vector data.

As described above, the input data includes at least the target cell lipid data set 10. As illustrated in FIG. 2, the target cell lipid data set 10 is a data set including at least a data element of a name of a lipid compound included in the cell membrane of the target cell and a data element related to a composition amount thereof. The data set 10 may include a plurality of sets of combinations of data elements of lipid composition ratios of the cell membrane of the target cell. Here, the plurality of sets of the combinations of the data elements refer to, for example, a combination of data of the lipid composition ratios obtained by applying different measurement methods to the same target cell, membrane composition data of the target cells prepared under different culture conditions, membrane composition data of the target cells of different lots, membrane composition of the target cells collected from different donors, and the like. By configuring the data set 10 with the plurality of sets of the combinations of the data elements, it is possible to acquire the target carrier composition data set 14 while considering a minute error regarding the lipid composition of the cell membrane of the target cell.

The composition amount of each lipid compound in the data set 10 may be set as a ratio based on a total of 100 parts by mass of the lipid compounds included in the cell membrane. The composition amount of each lipid compound may be a measured value of the lipid composition obtained by various known lipid quantification methods. For example, the target cell lipid data set 10 may be a measured value obtained by a measurement device outside the prediction device 1, or may be data determined with reference to a known literature. In a case where the data acquisition unit is provided as the input unit 2, the target cell lipid data set 10 is a measured value obtained by the data acquisition unit. Alternatively, as the composition amount of each lipid compound, a weight ratio or a molar ratio of each lipid compound with respect to all cell membrane constituent substances may be set. In this case, the data set 10 may include data regarding the cell membrane constituent substances (for example, membrane proteins) other than the lipid compound.

In order to more accurately grasp a feature indicated by the lipid composition of the cell membrane of the target cell, it is preferable that the number of types of lipid compounds included in the data set 10 is relatively large. The number of types of lipid compounds is at least 20 or more, and preferably 100 or more.

### - Designing Method

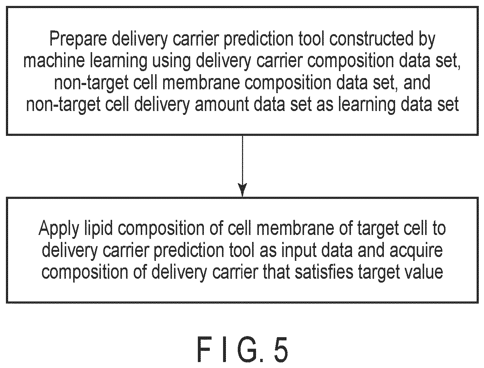

As illustrated in FIG. 5, as a procedure of a designing method for the delivery carrier, first, the delivery carrier prediction tool constructed by machine learning using, as learning data, delivery carrier composition data regarding a plurality of types of delivery carriers and indicating different component compositions, cell membrane composition data regarding lipid compositions of cell membranes of a plurality of types of non-target cells, and delivery amount data regarding delivery amounts of a delivered substance of the plurality of types of delivery carriers for the plurality of types of non-target cells is prepared. The lipid composition of the cell membrane of the target cell is then applied as the input data to the delivery carrier prediction tool to obtain the composition of the delivery carrier that satisfies the target value.

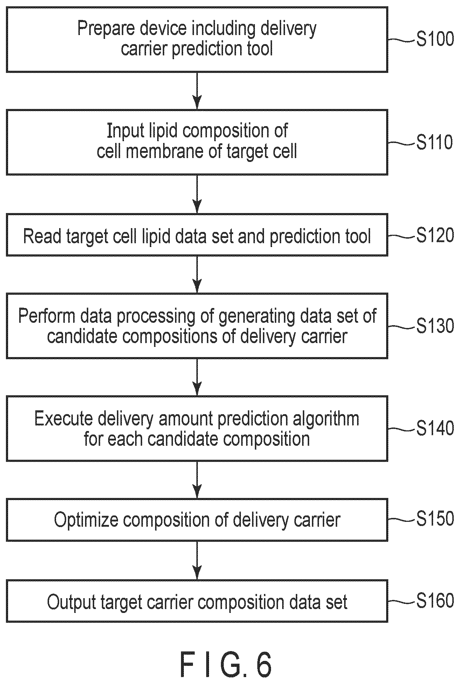

Further, the designing method using the above-described designing device will be described. As illustrated in FIG. 6, the designing device including the delivery carrier prediction tool is prepared (S100). Next, the lipid composition of the cell membrane of the target cell is input using the input unit 2 of the prediction device 1 (S110). Furthermore, a program group for executing the prediction tool 11 stored in the auxiliary storage unit 5 of the computation unit 3 is read and executed, thereby causing the computation unit 3 to execute each procedure (S120 to S150). Finally, the target carrier composition data set 14 that can achieve an excellent active agent delivery amount is acquired and output (S160), and the processing ends. Hereinafter, a content of each step (S100 to S160) will be described in detail.

In a step of inputting the lipid composition of the cell membrane of the target cell (S110), the target cell lipid data set 10 is input using the input unit 2 of the prepared prediction device 1. In a case where the computation unit 3 includes the auxiliary storage unit 5 and the main storage unit 6, the input data set 10 is stored in the auxiliary storage unit 5 or the main storage unit 6.

After step (S110), the computation unit 3 reads the target cell lipid data set 10 and the prediction tool 11 (step (S120)). For example, in a case where the computation unit 3 includes the auxiliary storage unit 5, the main storage unit 6, and the computation processing unit 7, the prediction tool stored in the auxiliary storage unit 5 is read into the main storage unit 6, and the data processing performed by the computation processing unit 7 is started.

After step (S110), the computation unit 3 performs the data processing of generating a data set of candidate compositions of the delivery carrier (step (S130)). The prediction tool is used to generate the candidate composition data. Specifically, the candidate composition data is generated by combining arbitrary components of multiple types of delivery carriers recorded in the data set used for machine learning in constructing the prediction tool and determining the composition amounts thereof. For example, in a case where a delivery carrier A and a delivery carrier B are stored in the data set used for machine learning in the construction of the prediction tool, a component X of the delivery carrier A and components Y and Z of the delivery carrier B are selected to generate a composition including the components X, Y, and Z with arbitrary composition amounts as the candidate composition.

After step (S130), the computation unit 3 applies the program 12 for executing the delivery amount prediction algorithm of the prediction tool to the generated data set of the candidate compositions of the delivery carrier, and performs the data processing (step (S140)). Examples of the delivery amount prediction algorithm include Lasso, Elastic Net, kernel ridge regression (KRR), Gaussian process regression (GPR), and a neural network.

After step (S140), the computation unit 3 optimizes the composition of the delivery carrier based on the predicted active agent delivery amount (step (S150)). Specifically, in step (S150), composition data of the delivery carrier for which a larger active agent delivery amount is predicted is selected from the candidate composition data set generated in step (S140) by using the program 13 that causes the computation unit 3 to execute the composition optimization algorithm.

For example, the program 13 (hereinafter, also referred to as a "composition optimization program") for executing the composition optimization algorithm includes: a candidate generation program that causes a computer to generate a plurality of candidate compositions of the delivery carrier; a delivery amount calculation program that causes the computer to calculate a predicted value of the active agent delivery amount of the delivery carrier of each candidate composition based on the plurality of generated candidate compositions and the lipid composition of the cell membrane of the target cell; and a determination program that causes the computer to compare each predicted value with the target value, determine whether or not each predicted value satisfies the target value, and select a candidate composition for which the predicted value satisfying the target value has been calculated.

The composition optimization algorithm is, for example, a method in which the candidate compositions are ranked based on the predicted value of the active agent delivery amount, and the candidate compositions of the delivery carrier that have the predicted values of the active agent delivery amount from the first rank to a predetermined rank are selected. That is, the number of pieces of composition data of the delivery carrier selected in step (S150) is not limited to one and may be plural depending on setting of the composition optimization algorithm.

Alternatively, the composition optimization algorithm is a method in which a predetermined target value is set, and a composition having a predicted value of the active agent delivery amount that is approximate to the target value is determined and selected as the delivery carrier having a large active agent delivery amount. Specifically, the program 13 is executed to cause the computation unit 3 to compare the calculated predicted value of the delivery amount with the target value, determine whether or not the predicted value of the delivery amount falls within an allowable range set to be approximate, and select the candidate composition data of the delivery carrier within the allowable range. The allowable range can be arbitrarily set, and for example, can be set within a range of about ±10% or ±5% with respect to the target value.

Alternatively, the composition optimization algorithm is a method in which the delivery carrier having the active agent delivery amount that is approximate to the target value is selected based on evaluation using an evaluation function. As the evaluation function for evaluating the degree of approximation between the target value and the active agent delivery amount, various known evaluation functions such as a mean absolute error, a mean squared error, a mean absolute percentage error, and a mean squared percentage error can be used. By using the evaluation function as an index for evaluating the active agent delivery amount approximate to the target value, the degree of influence on the active agent delivery amount due to a difference in composition of the delivery carrier can be grasped in more detail.

Alternatively, the composition optimization program 13 may be a program that searches for the composition data of the delivery carrier having the active agent delivery amount approximate to the target value by repeatedly performing the step (S130) of generating the candidates and the step (S140) of calculating the active agent delivery amount, and acquires the target carrier composition data set 14. For example, a range of about ±10% or ±5% with respect to the target value is set as the allowable range, and each of the steps (S130 and S140) is repeated until the calculated active agent delivery amount falls within the allowable range. That is, in step (S130) in a certain repetition, the candidates may be generated based on the index for evaluation of the active agent delivery amount calculated by the most recent or cumulative repetition.

The composition optimization algorithm that repeatedly performs steps (S130) and (S140) is a method using a generation model acquired by machine learning. Examples of the method of machine learning include Gaussian process regression, an optimization algorithm, multiple regression analysis, random forest, and a deep neural network (DNN) such as a recursive neural network (RNN), a generative adversarial network (GAN), or a conditional generative adversarial network (CGAN). Examples of the optimization algorithm include a genetic algorithm (GA), Bayesian optimization (BO), and a steepest descent method.

Here, an example of machine learning using the genetic algorithm will be described. First, a data set including multiple pieces of candidate composition data satisfying constraints by constraint conditions is generated. Next, the data set is set as an initial current-generation data set, and genetic manipulation (crossover and mutation) is performed on the composition data selected (roulette selection, tournament selection, elite selection, or the like) based on the evaluation index from the current-generation data set. Next, a next-generation data set in which a plurality of pieces of composition data generated by the genetic manipulation are accumulated is created, and the evaluation index is calculated for the created next-generation data set. Then, the created next-generation data set is regarded as the current-generation data set, and the selection, the genetic manipulation, the creation of the next-generation data set, and the calculation of the evaluation index are repeated. A data set of the final generation obtained by the repetition becomes a data set including composition data of an optimal solution. The data set including the composition data of the optimal solution is selected as the target carrier composition data set 14 acquired in steps (S130) to (S150).

After step (S150), the target carrier composition data set 14 selected by the computation unit 3 is output (step (S160)). In step (S160), the data processing of outputting the target carrier composition data set 14 is performed in the output unit 4. As the target carrier composition data set 14 is output by the output unit 4, the target carrier composition data set 14 can be acquired from the prediction device 1.

As described above, the prediction device 1 to which the target cell lipid data set 10 is input from the input unit 2 is configured to cause the computation unit 3 to read the prediction tool and the target cell lipid data set 10 stored in the auxiliary storage unit 5 into the main storage unit 6, cause the computation processing unit 7 to perform processing therefor to acquire the target carrier composition data set 14, and design the composition of the delivery carrier.

In such a prediction device 1, it is sufficient if the composition data acquired using the prediction device 1 is referred to when designing the delivery carrier. It is possible to significantly reduce the number of times experimental data is acquired by preparing the target cell and a wide variety of delivery carriers. Further, the preferred composition of the delivery carrier can be designed without an influence of human experience or skill. Therefore, such a configuration greatly contributes to efficiency of development work and improvement work for obtaining a desired delivery carrier.

Next, modifications of the designing device and the designing method according to the arrangement will be described.

### - Step of Constructing Prediction Tool

As a modification, the designing method for a delivery carrier may include, before step (S110), a step of constructing the prediction tool (step (S10)). In addition, the designing device for the delivery carrier may store a program for executing a machine learning algorithm for constructing the prediction tool in the computation unit 3.

More specifically, the prediction tool may be a plurality of programs constructed by machine learning using various data sets stored in the auxiliary storage unit 5 in the computation unit 3 of the prediction device 1. In other words, the predetermined program stored in the auxiliary storage unit 5 may be a program that reads various data sets stored in the auxiliary storage unit 5 and executes the machine learning algorithm in the computation unit 3, and the plurality of programs included in the prediction tool may be constructed by the program.

In step (S10), the data set used for machine learning of the prediction tool includes at least a data set regarding the active agent delivery amount for the non-target cell. Specifically, the data set regarding the active agent delivery amount for the non-target cell includes at least a data set 15 (hereinafter, referred to as a "non-target cell membrane composition data set") of the composition of the cell membrane of the non-target cell and a data set 16 (hereinafter, referred to as a "non-target cell delivery amount data set") of the active agent delivery amount observed by bringing the delivery carriers having a plurality of different compositions into contact with the non-target cell. That is, in a case where the prediction tool is constructed by machine learning in the prediction device 1, the auxiliary storage unit 5 may store the non-target cell membrane composition data set 15 and the non-target cell delivery amount data set 16 in addition to the target cell lipid data set 10.

In a case where the data acquisition unit is provided as the input unit 2 of the prediction device 1, the computation unit 3 (more specifically, the auxiliary storage unit 5) of the prediction device 1 prepared in step (S110) does not have to store the non-target cell membrane composition data set 15 and the non-target cell delivery amount data set 16. For example, it is sufficient if the data sets 15 and 16 are acquired by the data acquisition unit and stored in the auxiliary storage unit 5 after the prediction device 1 including the data acquisition unit as the input unit 2 is prepared. That is, in the prediction method, the data sets 15 and 16 may be introduced from the input unit 2 to the prediction device 1 as the input data.

The non-target cell membrane composition data set 15 includes at least the name or serial number of each lipid compound included in the cell membrane of the non-target cell and a data element related to the composition of each lipid compound. For example, each lipid compound is assigned with an individual serial number (labels of columns: No. 1 to No. 300 in the example of FIG. 3), and the non-target cell membrane composition data set 15 includes the data element representing the composition of each lipid compound. In addition, the non-target cell membrane composition data set 15 preferably includes a plurality of sets of data elements related to the composition of each lipid compound for each of a plurality of different types of non-target cells. In this case, the data element of the non-target cell membrane composition data set 15 also includes the name and identification number of the non-target cell. For example, the non-target cell membrane composition data set 15 includes data elements in which an individual serial number (labels of rows: No. 1 to No. 100 in the example of FIG. 3) is assigned to each non-target cell.

In order to acquire feature amount extraction data reflecting a feature indicated by the lipid composition of the cell membrane of the non-target cell and a tendency thereof, it is preferable that the number of types of lipid compounds and the number of types of non-target cells indicated by the data set 15 are relatively large. The number of types of lipid compounds indicated by the data elements of the data set 15 is at least 20 or more, and preferably 100 or more. In addition, the number of types of non-target cells indicated by the data elements of the data set 15 is at least 20 types or more, and preferably 50 types or more. Therefore, the data of the data set 15 is preferably data of 20 dimensions or more, preferably 50 dimensions or more.

In addition, the composition of each lipid compound in the data set 15 may have a proportion (weight ratio or molar ratio) relative to all the lipid compounds of the cell membrane, or may have a proportion relative to a specific lipid compound. Alternatively, the composition of each lipid compound in the data set 15 may have a proportion in all the cell membrane constituent substances, or may have a proportion set as a weight ratio or a molar ratio relative to the total amount of all the constituent substances included in the cell membrane. In this case, the data set 15 may include data regarding the cell membrane constituent substances (for example, membrane proteins) other than the lipid compound.

The composition of each lipid compound in the data set 15 may be a measured value of the lipid composition obtained by various known quantification methods. For example, the non-target cell membrane composition data set 15 may be a measured value obtained by a measurement device outside the prediction device 1, or may be data determined with reference to a known literature. In a case where the data acquisition unit is provided as the input unit 2, the data set 15 is a measurement value obtained by the data acquisition unit.

The non-target cell delivery amount data set 16 at least includes the data elements related to the serial numbers assigned to the compositions of the plurality of types of delivery carriers, the composition ratio of each component of the delivery carrier indicated by each serial number, the name or identification number of the non-target cell brought into contact with each delivery carrier, and the active agent delivery amount of each delivery carrier for each non-target cell. In addition, the non-target cell delivery amount data set 16 includes, for example, two data structures (I) and (II).

For example, the data structure (I) includes the data element of the serial number of the composition of the delivery carrier, the data element of the composition ratio of each component of the delivery carrier indicated by each serial number, and the data elements of the names or identification numbers of the components of all the delivery carriers. In a case where such a data structure (I) is referred to as a "delivery carrier composition data set", it can be understood that the non-target cell delivery amount data set 16 includes the delivery carrier composition data set.

The data structure (II) includes the data elements of the serial numbers of the compositions of the plurality of types of delivery carriers, the data element of the name and identification number of the non-target cell brought into contact with the delivery carrier, and the data element of the active agent delivery amount of each delivery carrier for the non-target cell.

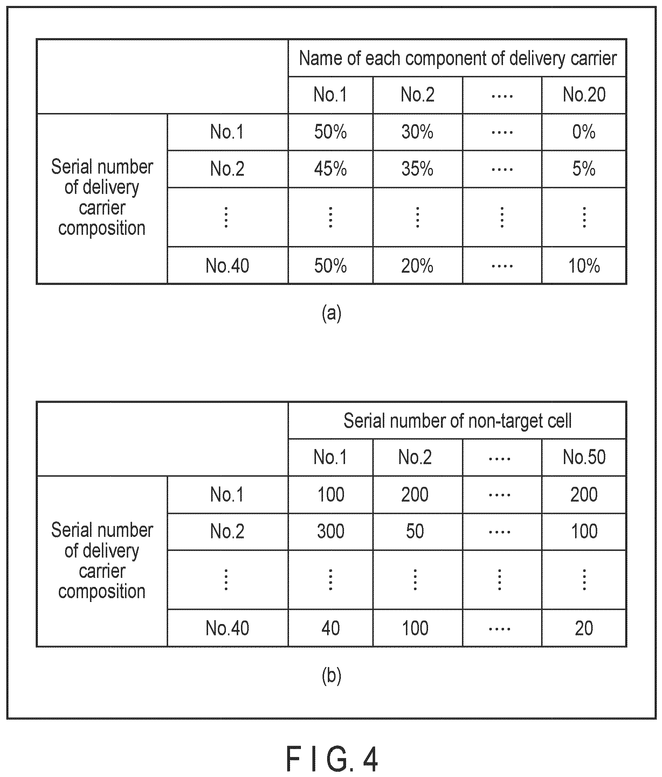

For example, as illustrated in part (a) of FIG. 4, the data structure (I) of the non-target cell delivery amount data set 16 includes the data element (labels of rows: No. 1 to No. 40 in part (a) of FIG. 4) of the serial number of the composition of the delivery carrier, the data element (labels of columns: No. 1 to No. 20 in part (a) of FIG. 4) of the name or identification number of each component of the delivery carrier, and the data element (numerical values in the table in part (a) of FIG. 4) of the composition ratio of each component of the delivery carrier.

In the data structure (I), the data element of the composition of each delivery carrier is a proportion of each component relative to the total amount, such as a weight ratio or mole fraction. The data illustrated in part (a) of FIG. 4 shows composition ratios of a plurality of types of lipid nanoparticles (No. 1 to No. 100). Since all the lipid nanoparticles of No. 1 to No. 100 in part (a) of FIG. 4 include a plurality of types of lipid compounds, the composition of the delivery carrier means the lipid composition ratio. Alternatively, for example, in a case where the lipid nanoparticle includes a component (for example, a transmembrane protein) other than the lipid compound, the weight ratio or mole fraction of each lipid compound may be calculated relative to the total weight or total substance amount of 100 of all the constituent substances, and set as the data structure (I) of the data set 16.

The data structure (I) of the data set 16 may include the data elements indicating information regarding each delivery carrier other than the components. For example, the data structure (I) may include, for each delivery carrier of each serial number, data of various physical values such as an average particle size of the delivery carrier, or data of a manufacturing method for the delivery carrier or environmental conditions at the time of manufacturing (for example, a temperature at the time of manufacturing).

The data element of the serial number of the composition of the delivery carrier of the data structure (II) is identical to the data element of the serial number of the composition of the delivery carrier of the data structure (I). That is, the data structure (II) includes the data element regarding the active agent delivery amount for the non-target cell in a case where the delivery carrier having the composition of each serial number illustrated in the data structure (I) is used. The data structure (II) preferably includes a plurality of sets of data of the active agent delivery amount for each of a plurality of different types of non-target cells. That is, the data structure (II) preferably includes the data elements including the names or identification numbers (labels of columns: No. 1 to No. 50 in the example of part (b) of FIG. 4) of the plurality of types of non-target cells.

The active agent delivery amount of the delivery carrier for the non-target cell is, for example, a measured value obtained by bringing the delivery carrier into contact with the non-target cell to deliver the active agent into the non-target cell, and further quantifying the active agent in the non-target cell. Such a measured value may be obtained by the data acquisition unit provided as the input unit 2 in the prediction device 1. Alternatively, the measured value may include a measured value acquired by a manufacturer of various preparations containing the delivery carrier in a manufacturing line such as a manufacturing test process and an inspection after manufacturing, a measured value acquired from laboratory data such as a large number of experiments in research and development, test results, and computer simulation results, data reported in known literatures, and the like.

The measured value of the active agent delivery amount for the non-target cell is a value obtained by various known quantification methods suitable for the type of the active agent to be used. Specifically, in a case where a nucleic acid is used as the active agent, a plurality of types of delivery carriers each encapsulating the nucleic acid and a plurality of types of non-target cells are prepared, and the non-target cell and the delivery carrier are brought into contact with each other in different combinations to deliver the nucleic acid into the non-target cell. Furthermore, by adding a fluorescent label that specifically reacts with the delivered nucleic acid to the non-target cell as a detection reagent, an amount of the nucleic acid introduced into each non-target cell is measured. The measured value obtained by such a quantitative method may be constructed as a database and set as the data set 16.

As the method of machine learning used for constructing the prediction tool, various machine learning algorithms can be used. Examples of the machine learning algorithm include Gaussian process regression, multiple regression analysis, random forest, and a deep neural network (DNN) such as a recursive neural network (RNN) or a generative adversarial network (GAN). By the machine learning, training for inferring the relevance between the composition of the delivery carrier, the lipid composition of the cell membrane of the cell into which the active agent is to be introduced by the delivery carrier, and the active agent delivery amount for the cell into which the active agent is to be introduced is performed.

### - Extraction of Feature Amount

As a further modification, the designing method for the delivery carrier may execute an algorithm (hereinafter, referred to as a "feature amount extraction algorithm") that reduces the number of dimensions of the composition of the component of the delivery carrier or the target cell and further extracts the feature amount. In addition, the designing device for the delivery carrier may store a program (hereinafter, referred to as a "feature amount extraction program") for executing the feature amount extraction algorithm in the computation unit.

In step (S130), a molecular structure of the delivery carrier is often relatively complex, and thus, a relevance between an overall molecular structure of the delivery carrier and a chemical property thereof is difficult to grasp, but each characteristic component in the delivery carrier is relatively easy to grasp. Therefore, by expressing the feature of the entire delivery carrier with the features of the components, it is possible to simply and accurately grasp the composition of the delivery carrier and the relevance thereof. Then, it is possible to efficiently acquire a delivery carrier composition satisfying a predetermined active agent delivery amount by using the non-target cell delivery amount data set acquired using the grasped relevance.

In addition, an excessive computation load may be applied to the construction of the prediction tool in step (S10), the generation of the candidate compositions by using the prediction tool in steps (S110) to (S130), and the acquisition of the target carrier composition data set. For example, in general, there are several hundred types of lipid compounds included in the cell membrane. In addition, since the composition of the delivery carrier can be artificially designed, there may be many composition patterns. In such a case, the data elements of each data set and the number of dimensions of the data set described above become enormous, and a large amount of calculation resources are required to handle each data set described above.

On the other hand, in a case where the number of types of lipid compounds of the cell membrane and the number of composition patterns of the delivery carrier are very large, information regarding the components of the lipid compound and the delivery carrier that hardly contribute to the active agent delivery amount of the delivery carrier is often included. It is not necessary to apply the computation in step (S10) or steps (S110) to (S130) to such information, and the computation can be omitted.

Therefore, in steps (S10) and (S110), it is preferable to exclude information with low necessity from each data set used for computation. Specifically, the computation load may be reduced by calculating the feature amount. The setting of the condition can be defined by the feature amount extraction program.

Specifically, the feature amount extraction program is executed to reduce the number of dimensions of the target cell lipid data set 10, and the data processing is further performed by the computation unit 3 to acquire a data set 17 (hereinafter, referred to as a "target cell lipid feature amount data set") of the feature amount of the lipid composition of the cell membrane of the target cell. Using the data set 17 in place of the data set 10 for subsequent calculations (for example, prediction of the active agent delivery amount) can save calculation resources. The acquired data set 17 may be stored in the auxiliary storage unit 5 in the computation unit 3. Further, the feature amount extraction program is executed and the data processing is performed by the computation unit 3 to acquire a data set 18 (hereinafter, referred to as a "non-target cell lipid feature amount data set") of the feature amount of the lipid composition of the cell membrane of the non-target cell and use the data set 18 for subsequent calculations in place of the data set 16. In addition, the acquired data set 18 may be configured to be stored in the auxiliary storage unit 5.

The feature amount extraction program may be stored in the auxiliary storage unit 5 or may be included as one of a program group included in the prediction tool. The feature amount extraction algorithm may be an algorithm for extracting the feature amount of the composition having the reduced number of dimensions, that is, an algorithm in which the reduction in the number of dimensions and the extraction of the feature amount are integrated with each other, or an algorithm in which the reduction in the number of dimensions and the extraction of the feature amount are separate from each other. Examples of the feature amount extraction algorithm include a variational autoencoder (VAE), a t-distributed stochastic neighbor embedding (t-SNE) method, gradient boosting (for example, XGBoost), and a feature amount selection method using random forest (for example, Boruta) .

In order to reduce the number of dimensions, it is preferable to reduce the number of dimensions by setting a certain constraint condition and generating a component satisfying the condition as a data set. For example, in a case where the number of dimensions of the data element of the lipid composition of the cell membrane of the target cell of the data set 15 is reduced, a program may perform the reduction only for a main component of the lipid composition of the cell membrane. Specifically, a plurality of types of lipids in the lipid composition of the cell membrane of the target cell may be regarded as the main components in descending order of the composition amount. Alternatively, in the generation of the composition data of the candidate delivery carriers, in a case where there is a desired substance that is desired to be the main component of the delivery carrier, data of the candidate composition with the substance as a constraint condition may be generated.

Further, the constraint condition is not limited to the above. For example, when manufacturing conditions and the like are included in the data set 15 and a specific manufacturing condition is desired, the specific manufacturing condition can be used as the constraint condition. In this way, by constraining the manufacturing conditions, it is possible to consider an influence of the manufacturing conditions of the delivery carrier on the delivery amount in a process of acquiring the target carrier composition data set. As a result, it is possible to easily grasp the manufacturing conditions for manufacturing the delivery carrier.

In the designing method, the program for executing the feature amount extraction algorithm may be constructed by the machine learning algorithm simultaneously with the step of constructing the prediction tool (S10).

### - Input of Training Data

As a further modification, the designing method for the delivery carrier may provide a data set of an active agent delivery amount acquired by introducing a delivery carrier having a known composition into a target cell to the prediction tool as the training data via the input unit 2. That is, the prediction device may store a program that causes the computer to correct the composition of the delivery carrier with the training data. The provision of the training data aims to correct and more accurately predict target carrier composition data obtained by the prediction tool. The composition of the delivery carrier of the training data may be different from the composition of the delivery carrier of the data element of the non-target cell delivery amount data set 16.

More specifically, the training data may be applied to a composition optimization algorithm that repeatedly performs steps (S130) and (S140). Since the correction is performed using the training data every time each of the above procedures is repeated, the target carrier composition data can be more accurately predicted.

While certain arrangements have been described, these arrangements have been presented by way of example only, and are not intended to limit the scope of the claims. Indeed, the method, program and device described herein may be embodied in a variety of other forms; furthermore, various omissions, substitutions and changes in the form of the method, program and device described herein may be made.

The arrangements as described above include clauses below.

### Clause 1

A designing method for predicting a composition of a delivery carrier that satisfies a target value of an active agent delivery amount for a target cell, the method characterized by comprising:
preparing a delivery carrier prediction tool constructed by machine learning using, as learning data sets, a delivery carrier composition data set regarding a plurality of types of delivery carriers having different component compositions, a non-target cell membrane composition data set regarding lipid compositions of cell membranes of a plurality of types of cells other than the target cell, and a non-target cell delivery amount data set regarding delivery amounts of a delivered substance of the types of delivery carriers for the types of cells other than the target cell; and
acquiring, using the delivery carrier prediction tool, the composition of the delivery carrier that satisfies the target value based on the lipid composition of the cell membrane of the target cell.

### Clause 2

The method according to Clause **1,** characterized in that the acquiring includes: generating a plurality of candidate compositions of the delivery carrier; calculating a predicted value of the active agent delivery amount of the delivery carrier having each of the candidate compositions from the generated candidate compositions and the lipid composition of the cell membrane of the target cell; and comparing the predicted value with the target value to determine whether or not the predicted value is the composition of the delivery carrier that satisfies the target value.

### Clause 3

The method according to Clause **1,** characterized in that the preparing of the delivery carrier prediction tool includes:
preparing the delivery carrier composition data set, the non-target cell membrane composition data set, and the non-target cell delivery amount data set, and
performing machine learning using, as the learning data sets, the delivery carrier composition data set, the non-target cell membrane composition data set, and the non-target cell delivery amount data set to construct the delivery carrier prediction tool.

### Clause 4

The method according to Clause 3, characterized in that the machine learning is Gaussian process regression, multiple regression analysis, random forest, or a deep neural network (DNN).

### Clause 5

The method according to Clause 1, characterized by further comprising extracting a feature amount of the lipid composition of the cell membrane of the target cell.

### Clause 6

The method according to Clause 5, characterized in that the feature amount is extracted by a feature amount selection method using a variational autoencoder (VAE), a t-distributed stochastic neighbor embedding (t-SNE) method, gradient boosting, or random forest.

### Clause 7

The method according to Clause 1, characterized by further comprising:
providing, as training data, a data set of an active agent delivery amount obtained by introducing a delivery carrier having a known composition into the target cell and performing measurement to the delivery carrier prediction tool; and
correcting the composition of the delivery carrier with the training data.

### Clause 8

A program for causing a computer to predict a composition of a delivery carrier that satisfies a target value of an active agent delivery amount for a target cell,
the program characterized by being constructed by machine learning using, as learning data sets, a delivery carrier composition data set regarding a plurality of types of delivery carriers having different component compositions, a non-target cell membrane composition data set regarding lipid compositions of cell membranes of a plurality of types of cells other than the target cell, and a non-target cell delivery amount data set regarding delivery amounts of a delivered substance of the types of delivery carriers for the types of cells other than the target cell, and
causing the computer to predict the composition of the delivery carrier that satisfies the target value of the active agent delivery amount for the target cell based on a data set of the lipid composition of the cell membrane of the target cell.

### Clause 9

The program according to Clause 8, characterized in that the program includes a composition optimization program, and
the composition optimization program includes:
a candidate generation program that causes the computer to generate a plurality of candidate compositions of the delivery carrier;
a delivery amount calculation program that causes the computer to calculate a predicted value of the active agent delivery amount of the delivery carrier having each of the generated candidate compositions and the lipid composition of the cell membrane of the target cell; and
a determination program that causes the computer to compare each of the predicted values with the target value, determine whether each of the predicted values satisfies the target value, and select the candidate composition for which the predicted value satisfying the target value has been calculated.

### Clause 10

The program according to Clause 9, characterized in that the composition optimization program causes the computer to repeatedly execute the candidate generation program, the delivery amount calculation program, and the determination program until the predicted value satisfies the target value.

### Clause 11

The program according to Clause 10, characterized in that the composition optimization program of the repeated execution is a genetic algorithm, Bayesian optimization, or a steepest descent method.

### Clause 12

The program for causing a computer to perform machine learning for constructing the program according to Clause 8, characterized in that
the machine learning causing the computer to infer a relevance between the delivery carrier composition data set, the non-target cell membrane composition data set, and the non-target cell delivery amount data set by using, as the learning data sets, the delivery carrier composition data set, the non-target cell membrane composition data set, and the non-target cell delivery amount data set.

### Clause 13

The program according to Clause 12, characterized in that the machine learning is Gaussian process regression, multiple regression analysis, random forest, or a deep neural network (DNN).

### Clause 14

The program according to Clause 9, characterized in that the computer is caused to execute a feature amount extraction program for extracting a feature amount of the lipid composition of the cell membrane of the target cell, and calculate the predicted value of the delivery carrier having each of the candidate compositions by using the obtained feature amount.

### Clause 15

The program according to Clause 14, characterized in that the feature amount is extracted by a feature amount selection method using a variational autoencoder (VAE), a t-distributed stochastic neighbor embedding (t-SNE) method, gradient boosting, or random forest.

### Clause 16

The program according to Clause 12, characterized in that
the computer is caused to execute a feature amount extraction program for extracting respective feature amounts of the delivery carrier composition data set, the non-target cell membrane composition data set, and the non-target cell delivery amount data set, and
the machine learning causes the computer to infer a relevance between the respective feature amounts by using the respective feature amounts.

### Clause 17

The program according to Clause 16, characterized in that the feature amount is extracted by a feature amount selection method using a variational autoencoder (VAE), a t-distributed stochastic neighbor embedding (t-SNE) method, gradient boosting, or random forest.

### Clause 18

The program according to Clause 9, characterized in that the computer is caused to correct the composition of the delivery carrier with training data.

### Clause 19

A device (1) for predicting a composition of a delivery carrier that satisfies a target value of an active agent delivery amount for a target cell, the device characterized by comprising:
an input unit (2) configured to receive a lipid composition of a cell membrane of the target cell from outside of the device;
a computation unit (3) configured to calculate the composition of the delivery carrier that satisfies the target value of the active agent delivery amount for the target cell based on the lipid composition of the cell membrane of the target cell transmitted from the input unit by using a delivery carrier prediction tool (11) constructed by machine learning using, as learning data sets, a delivery carrier composition data set regarding a plurality of types of delivery carriers having different component compositions, a non-target cell membrane composition data set regarding lipid compositions of cell membranes of a plurality of types of cells other than the target cell, and a non-target cell delivery amount data set regarding delivery amounts of a delivered substance of the types of delivery carriers for the types of cells other than the target cell; and
an output unit (4) configured to display a computation result of the computation unit.

## Claims

1. A designing method for predicting a composition of a delivery carrier that satisfies a target value of an active agent delivery amount for a target cell, the method **characterized by** comprising:
preparing a delivery carrier prediction tool constructed by machine learning using, as learning data sets, a delivery carrier composition data set regarding a plurality of types of delivery carriers having different component compositions, a non-target cell membrane composition data set regarding lipid compositions of cell membranes of a plurality of types of cells other than the target cell, and a non-target cell delivery amount data set regarding delivery amounts of a delivered substance of the types of delivery carriers for the types of cells other than the target cell; and
acquiring, using the delivery carrier prediction tool, the composition of the delivery carrier that satisfies the target value based on the lipid composition of the cell membrane of the target cell.

2. The method according to claim 1, **characterized in that** the acquiring includes:
generating a plurality of candidate compositions of the delivery carrier;
calculating a predicted value of the active agent delivery amount of the delivery carrier having each of the candidate compositions from the generated candidate compositions and the lipid composition of the cell membrane of the target cell; and
comparing the predicted value with the target value to determine whether or not the predicted value is the composition of the delivery carrier that satisfies the target value.

3. The method according to claim 1, **characterized in that** the preparing of the delivery carrier prediction tool includes:
preparing the delivery carrier composition data set, the non-target cell membrane composition data set, and the non-target cell delivery amount data set, and
performing machine learning using, as the learning data sets, the delivery carrier composition data set, the non-target cell membrane composition data set, and the non-target cell delivery amount data set to construct the delivery carrier prediction tool.

4. The method according to claim 1, **characterized by** further comprising extracting a feature amount of the lipid composition of the cell membrane of the target cell.

5. The method according to claim 1, **characterized by** further comprising:
providing, as training data, a data set of an active agent delivery amount obtained by introducing a delivery carrier having a known composition into the target cell and performing measurement to the delivery carrier prediction tool; and
correcting the composition of the delivery carrier with the training data.

6. A program for causing a computer to predict a composition of a delivery carrier that satisfies a target value of an active agent delivery amount for a target cell,
the program **characterized by** being constructed by machine learning using, as learning data sets, a delivery carrier composition data set regarding a plurality of types of delivery carriers having different component compositions, a non-target cell membrane composition data set regarding lipid compositions of cell membranes of a plurality of types of cells other than the target cell, and a non-target cell delivery amount data set regarding delivery amounts of a delivered substance of the types of delivery carriers for the types of cells other than the target cell; and
causing the computer to predict the composition of the delivery carrier that satisfies the target value of the active agent delivery amount for the target cell based on a data set of the lipid composition of the cell membrane of the target cell.

7. The program according to claim 6, **characterized in that** the program includes a composition optimization program, and
the composition optimization program includes:
a candidate generation program that causes the computer to generate a plurality of candidate compositions of the delivery carrier;
a delivery amount calculation program that causes the computer to calculate a predicted value of the active agent delivery amount of the delivery carrier having each of the generated candidate compositions and the lipid composition of the cell membrane of the target cell; and
a determination program that causes the computer to compare each of the predicted values with the target value, determine whether each of the predicted values satisfies the target value, and select the candidate composition for which the predicted value satisfying the target value has been calculated.

8. The program according to claim 7, **characterized in that** the composition optimization program causes the computer to repeatedly execute the candidate generation program, the delivery amount calculation program, and the determination program until the predicted value satisfies the target value.

9. The program according to claim 8, **characterized in that** the composition optimization program of the repeated execution is a genetic algorithm, Bayesian optimization, or a steepest descent method.

10. The program for causing a computer to perform machine learning for constructing the program according to claim 6, **characterized in that**
the machine learning causing the computer to infer a relevance between the delivery carrier composition data set, the non-target cell membrane composition data set, and the non-target cell delivery amount data set by using, as the learning data sets, the delivery carrier composition data set, the non-target cell membrane composition data set, and the non-target cell delivery amount data set.

11. The program according to claim 7, **characterized in that** the computer is caused to execute a feature amount extraction program for extracting a feature amount of the lipid composition of the cell membrane of the target cell, and calculate the predicted value of the delivery carrier having each of the candidate compositions by using the obtained feature amount.

12. The program according to claim 10, **characterized in that**
the computer is caused to execute a feature amount extraction program for extracting respective feature amounts of the delivery carrier composition data set, the non-target cell membrane composition data set, and the non-target cell delivery amount data set, and
the machine learning causes the computer to infer a relevance between the respective feature amounts by using the respective feature amounts.

13. The program according to claim 7, **characterized in that** the computer is caused to correct the composition of the delivery carrier with training data.

14. A device (1) for predicting a composition of a delivery carrier that satisfies a target value of an active agent delivery amount for a target cell, the device **characterized by** comprising:
an input unit (2) configured to receive a lipid composition of a cell membrane of the target cell from outside of the device;
a computation unit (3) configured to calculate the composition of the delivery carrier that satisfies the target value of the active agent delivery amount for the target cell based on the lipid composition of the cell membrane of the target cell transmitted from the input unit (2) by using a delivery carrier prediction tool (11) constructed by machine learning using, as learning data sets, a delivery carrier composition data set regarding a plurality of types of delivery carriers having different component compositions, a non-target cell membrane composition data set regarding lipid compositions of cell membranes of a plurality of types of cells other than the target cell, and a non-target cell delivery amount data set regarding delivery amounts of a delivered substance of the types of delivery carriers for the types of cells other than the target cell; and
an output unit (4) configured to display a computation result of the computation unit (3).
